# EUROPEAN PATENT APPLICATION

(11) **EP 4 439 495 A1**
(43) Date of publication of application: **02.10.2024**
(21) Application number: 23165669.5
(22) Date of filing: 30.03.2023
(51) Int. Cl.: G06V 40/14, A61N 5/06

(54) **OPTICAL SYSTEM FOR A BODY TREATMENT DEVICE**

(71) Applicant: Koninklijke Philips N.V., 5656 AG Eindhoven (NL)
(72) Inventor: BEREZHNOY, Igor, 5656AG Eindhoven (NL); KULKARNI, Nikhil Vikram, 5656AG Eindhoven (NL); MENDOZA, Jose Luis Diaz, 5656AG Eindhoven (NL); PATIL, Ravindra Balasaheb, 5656AG Eindhoven (NL); JUAREZ URIZAR, Oscar Ricardo, 5656AG Eindhoven (NL)
(74) Representative: Philips Intellectual Property & Standards

(57) **Abstract**

A sensing system for use with a body care or body treatment device, such as a personal care device. The sensing system may be integrated in the device. The sensing system acquires optical sensing data of a tissue area proximal to the body care device during use, processes the sensing data to extract a representation of a superficial vein or blood vessel pattern at the tissue area and uses the representation of the vein pattern to infer information about a class of user (e.g. demographic class) or a body location to which the sensed tissue area belongs. Optionally, treatment-related information can be generated using this information. This may be an operational parameter setting, or may be monitoring information such as coverage information.

## Description

### FIELD OF THE INVENTION

The present invention relates to the field of body treatment devices, particularly handheld body treatment devices.

### BACKGROUND OF THE INVENTION

This invention relates to body care devices, in particular those which employ a hand-held unit having an operative treatment area which is passed over body surfaces of a user to perform a body-treatment function. Examples of such devices include grooming devices such as hair removal devices, shavers, and beard trimmers. One category of hair removal devices is Intense Pulsed Light (IPL) hair removal devices. Additionally, there are other devices designed for skin treatment, such as optical skin treatment devices.

### SUMMARY OF THE INVENTION

It has been recognized by the inventors that, in the context of any device which is moveable, e.g. hand-operated, and so can be applied to a variety of different particular body areas and/or used by a variety of different users, it would be a valuable to permit variation in functionality in dependence upon the person or body location to which the device is being applied. A standard way of implementing this is to provide a user control interface which permits a user to manually adjust settings of the device. However, it has been the recognition of the inventors that it would be advantageous to enable automated configuration of device settings. The inventors have recognized that to facilitate this, a means for automated identification or classification of a user or body part to which a device is being applied would be of value. Indeed, this functionality would have value beyond settings configuration, e.g. for performance or coverage monitoring.

It has further been recognized by the inventors that it would be advantageous to enable automated tracking of device coverage over a relevant body area to enable insights to be provided to a user regarding potential missed areas and areas which have been overexposed.

The invention is defined by the claims.

According to examples in accordance with an aspect of the invention, there is provided an optical sensing system for a body treatment device. The system comprises a vein visualization sub-system for integration in the body treatment device, for detecting a pattern of a user's superficial veins. The vein visualization subsystem comprises an optical arrangement which includes: a light source for directing light into surface tissue of the user during use of the body treatment device, and an optical detector for detecting a pattern of light reflected from the surface tissue having a vein pattern represented therein. The system further comprises a classification module for application to an output from the vein visualization sub-system to provide one or more classifications of a biological structure or region to which a vein pattern represented in the detected pattern of light belongs. The system further comprises a processing module adapted to generate treatment-related information using an output from the classification module for at least one detected vein pattern.

The detected light pattern may for example be an image.

The inventors have recognized that an approach to addressing the above-mentioned problems may be arrived at by optically scanning skin as a device is being used and taking advantage of predictable variations in the patterning of superficial vein structures at different areas of the body and for users of different demographic categories.

As will be discussed further below, it is known from the literature that superficial vein patterns are unique to each part of the body, e.g. leg, arm, feet, hands, etc. It is also known from the literature that vein patterns vary with age and sex of the individual. The inventors thus propose to use this predictable variation in superficial vein patterns as a function of body part and demographics to identify the demographic category of person or the body part to which the device is being applied. In other words, the broader biological structure or region thereof which is scanned by the optical system is classified. This classification can subsequently be used to determine treatment-related information. This may for example comprise an appropriate operational setting for the device, or it may comprise monitoring information, such as body coverage tracking information. Thus, it is proposed to use vein patterns to automatically infer locational context information as to whom or to what area the device is being applied.

More concretely, in some embodiments, the one or more classifications generated by the classification module may comprise any one or more of: an identification of a body part to which the vein pattern belongs; a classification of one or more demographic characteristics of the individual to which the vein pattern belongs, for example age and/or sex; and/or an identification of a unique skin patch within which the vein pattern is situated.

In some embodiments, the vein visualization subsystem may further comprise a pattern extraction module for extracting a representation of a vein pattern from the detected pattern of light. The classification module may be adapted to operate on the extracted representation of the vein pattern.

In some embodiments, the representation of the vein pattern comprises a vein pattern mask.

In some embodiments, the pattern extraction module may be a trained neural network which is trained to extract the vein pattern mask from a detected light pattern. The neural network may be trained on training data which comprises images depicting different vein pattern masks, each annotated with a ground truth vein pattern mask.

In some embodiments, the classification module may comprise a trained neural network. By way of one example, this may be a convolution neural network, for example with U-net architecture. In some embodiments the neural network employed by the classification module may be trained to receive a vein pattern mask as input as to generate at least one of the relevant one or more classifications as output.

By way of example, the trained neural network employed by the classification module may be a network trained using training data which comprises vein pattern masks annotated with ground truth information indicative of a biological structure or region to which a vein pattern represented in the vein pattern mask belongs. For example, the ground truth information may be indicative of one or more of: a body part to which the vein pattern belongs; age of the individual to which the vein pattern belongs; sex of the individual to which the vein pattern belongs, and a unique skin patch identifier of the skin patch to which the vein pattern belongs.

In some embodiments, the classification module is for integration within the body treatment device.

In some embodiments, the classification module comprises a memory storing the neural network and a processor for controlling application of the neural network.

In some embodiments, the neural network may be quantized before integration in the device to reduce the size of the model, thereby enabling it to be deployed by hardware having small memory and processing capacity.

In some embodiments, the treatment related information comprises electronic control instructions for adjusting an operational parameter of the body treatment device in dependence upon the classification.

In some embodiments, the operational parameter comprises selection between available treatment modes, wherein a first mode is a shaving mode, and a second mode is a hair removal mode.

In some embodiments, the body treatment device is a shaving device and wherein the operational parameter comprises a cutting speed/rate of a cutting mechanism of the shaving device. For example, a higher cutting rate may be set when the device is being applied to the face compared to when the device is being applied to the chest.

In some embodiments, the body treatment device is an Intense Pulsed Light (IPL) hair removal device and wherein the operational parameter comprises an intensity level of pulsed light generated by the device.

As mentioned above, in addition to or instead of using the classification to configure an operational setting of the device, the classification can be used to track coverage of the body by the device during a treatment session.

In this regard, in some embodiments, the classifier module may be adapted to generate a classification comprising an identification of a unique skin patch within which the vein pattern is situated. In some embodiments, the system further comprises a coverage tracking module comprising one or more processors adapted to, during a body treatment session, receive an indicator of each detected unique skin patch; and store a record of each skin patch detection in a memory module to compile a body treatment session history. In this case, the treatment-related information previously referred to may comprise the body treatment session history.

In some embodiments, the processing module is adapted to identify from the treatment session history any unique skin patches for which multiple detections are recorded in the session history. The treatment-related information in this case may include an indication of each unique skin patch for which multiple detections are recorded. This allows for identifying skin areas which have been covered more than once during a given treatment session.

In some embodiments, the processing module may be comprised by an analytics module. The analytics module may be adapted to retrieve the body treatment session history for at least one body treatment session from the memory module. The analytics module may be further adapted to access a data structure recorded in a datastore which records a set of known or assumed unique skin patches comprised by the user. In this case, the treatment-related information may comprise coverage feedback indicative of coverage and/or non-coverage of different of the unique skin patches during the treatment session.

In some embodiments, the data structure recorded in the datastore may comprise a body map comprising an indicator of a bodily location of each of the set of unique skin patches.

In some embodiments, the coverage feedback may comprise a graphical representation of the body map, including a visual indication of coverage and/or non-coverage of each of the unique skin patches. Optionally, the processing module may be adapted to communicate the coverage feedback to a user interface or to a mobile computing device.

Consistent at least with any of the above-described embodiments, the system may also include one or more of the following features.

In some embodiments, the light source may be an infrared (IR) light source.

In some embodiments, the light source comprises an array of lighting elements, for example an elongate or linear array of lighting elements.

In some embodiments, the optical detector comprises an array of light detection elements, for example an elongate or linear array of light detection elements.

In some embodiments, the light source comprises an elongate or linear array of lighting elements and the optical detector comprises an elongate or linear array of light detection elements being parallel with the elongate or linear array of lighting elements.

This provides a structurally efficient arrangement wherein, by moving the optical arrangement in a direction perpendicular to the linear arrays, illumination and detection can simultaneously be performed.

In some embodiments, the vein visualization sub-system further comprises: a proximity sensor for detecting a proximity distance between the optical detector and the surface tissue of the user. In some embodiments, the vein visualization sub-system further comprises a patch normalization module for applying a positive or negative enlargement of the detected light pattern of a magnitude determined in dependence on the sensed proximity distance in order to normalize each detected light pattern size to a standardized size.

Another aspect of the invention is a body-treatment device comprising the optical sensing system according to any example or embodiment described in this disclosure.

In some embodiments, the body treatment device is an Intense Pulsed Light (IPL) hair removal device or the body treatment device is a shaver or other mechanical hair removal device.

In some embodiments, the vein visualization subsystem comprises an optical input/output area arranged for being applied against the skin of the user during use of the device and wherein the light source and the optical detector emit light and detect light via the optical input/output area. For example, this may comprise an optical window. This area may form a tissue contact area.

In some embodiments, the body treatment device includes an operative area at a surface of the device for engaging with tissue to perform a body treatment function, and wherein the optical detection system includes a plurality of the optical arrangements, including at least one optical arrangement disposed on one side of the operative area and at least a second optical arrangement disposed on an opposite side of the operational area. This allows for obtaining directional information.

Another aspect of the invention provides an optical sensing method for use during application of a body-treatment device to the body of a user, comprising: detecting a vein pattern of a user's superficial veins, wherein the detecting comprises: directing light into surface tissue of the user during use of the body treatment device; and detecting a pattern of light reflected from the surface tissue responsive to the directing of the light. The method further comprises applying a classification module to provide one or more classifications of a biological structure or region thereof to which a vein pattern represented in the detected pattern of light belongs. The method further comprises generating treatment-related information using an output from the classification module for at least one detected vein pattern.

In some embodiments, the classification module comprises a trained neural network, and wherein the method includes a training procedure for training the neural network comprising training the network using training data which comprises vein pattern masks annotated with ground truth information indicative of a biological structure or region to which a vein pattern represented in the vein pattern mask belongs. For example, the ground truth information may be indicative of one or more of: a body part to which the vein pattern belongs; age of the individual to which the vein pattern belongs; sex of the individual to which the vein pattern belongs; and a unique skin patch identifier of the skin patch to which the vein pattern belongs.

In some embodiments, the method further comprises quantizing the neural network after training. The neural network may be quantized before integration in the device to device the size of the model, thereby enabling it to be deployed by hardware having small memory and processing capacity.

Another aspect of the invention is a computer program product comprising machine-executable instructions configured, when executed by a processor, to cause the processor to: control detection of a vein pattern of a user's superficial veins by:
communicating with a light source to control the light source to direct light into surface tissue of the user during use of the body treatment device; and
communicating with an optical detector to control the optical detector to detect a pattern of light reflected from the surface tissue responsive to the directing of the light;
apply a classification module to provide one or more classifications of a biological structure or region thereof to which a vein pattern represented in the detected pattern of light belongs; and
generate treatment-related information using an output from the classification module for at least one detected vein pattern.

Another aspect of the invention is a method of training an artificial neural network comprising: receiving training data, wherein the training data comprises a plurality of training data entries, each training data entry comprising a vein pattern mask annotated with ground truth information indicative of a biological structure or region to which a vein pattern represented in the vein pattern mask belongs; and training the artificial neural network using the training data.

In some embodiments, the the ground truth information is indicative of one or more of: a body part to which the vein pattern belongs, an age of the individual to which the vein pattern belongs, a sex of the individual to which the vein pattern belongs, and a unique skin patch identifier of the skin patch to which the vein pattern belongs.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates example vein patterns detectable on an arm and on the feet;
Fig. 2 illustrates example images acquired of a vein pattern on a forearm of each of a plurality of different subjects, of different age/sex categories;
Fig. 3 outlines steps of an example method in accordance with one or more embodiments of the invention;
Fig. 4 is a block diagram of an example system in accordance with one or more embodiments of the invention;
Fig. 5 is a block diagram of an example system in accordance with one or more embodiments of the invention in which a classifier module acts directly on detected light patterns or imaged;
Fig. 6 is a block diagram of an example system in accordance with one or more embodiments of the invention in which a classifier module acts on vein pattern masks extracted from detected light patterns or images by a pattern extraction module;
Figs.7 and 8 schematically illustrate example body treatment devices having an optical sensing system according to one or more embodiments of the invention integrated therein;
Fig. 9 schematically illustrates a structure of optical components of an example optical sensing system according to one or more embodiments of the invention;
Fig. 10 schematically illustrates an example vein pattern mask derived from measured light pattern data;
Fig. 11 schematically outlines at least a portion of the processing flow according to one or more embodiments;
Fig. 12 schematically outlines a processing flow for configuring an operation parameter setting based on a classification in accordance with one or more embodiments;
Fig. 13 schematically outlines a processing flow in accordance with a specific set of embodiments in which an operation mode of a body care device is varied in dependence upon a classification;
Fig. 14 schematically outlines a processing flow in accordance with a specific set of embodiments in which a motor speed of a hair cutting device such as a shaver is adjusted in dependence upon a classification;

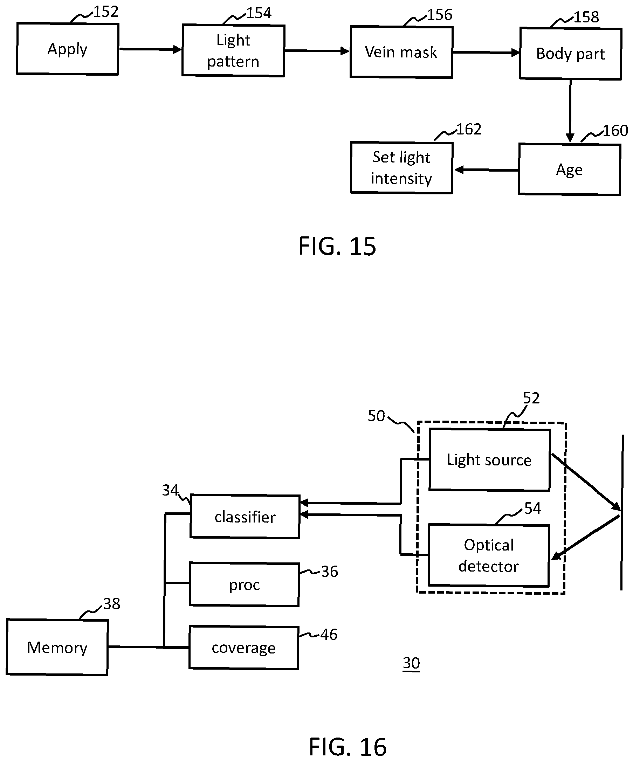

Fig. 15 schematically outlines a processing flow in accordance with a specific set of embodiments in which an intensity level of a treatment light of an optical hair removal device is adjusted in dependence upon a classification; and
Fig. 16 is a block diagram of a further example system in accordance with one or more embodiments of the invention in which a coverage tracking module is included.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a sensing system for use with a body care or body treatment device, such as a personal care device. The sensing system may be integrated in the device. The sensing system acquires optical sensing data of a tissue area proximal to the body care device during use, processes the sensing data to extract a representation of a superficial vein or blood vessel pattern at the tissue area and uses the representation of the vein pattern to infer information about a class of user (e.g. demographic class) or a body location to which the sensed tissue area belongs. Optionally, treatment-related information can be generated using this information. This may be an operational parameter setting, or may be monitoring information such as coverage information.

Thus, it is proposed to use vein visualization techniques to analyze optical sensor data, e.g. images, of the user's superficial veins.

According to at least one set of embodiments, it is proposed to use an image classification model, e.g. an AI-based image classification model, to determine which area of the body the vein pattern belongs to and/or the demographic classification of the person to which vein pattern belongs.

A brief background to superficial vein patterns will now be presented.

A superficial vein is a vein that is close to the surface of the skin. This is different to deep veins which are more remote from the surface. While deep veins are typically each paired with a corresponding artery of the same name, this is not true of superficial veins.

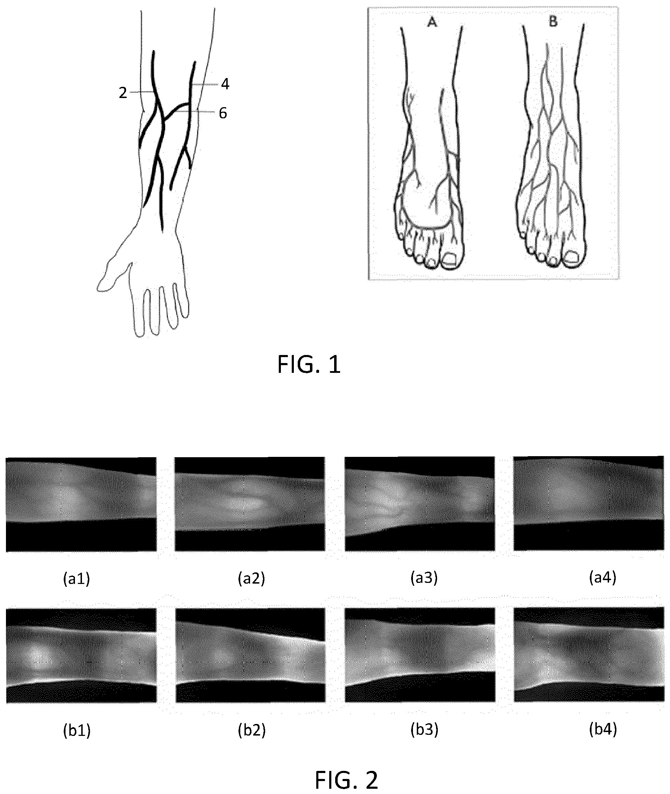

Fig. 1 schematically illustrates superficial vein patterns of the forearm and the feet. For example, in relation to the forearm, the cephalic vein 2, the basilic vein 4 and the median cubital vein 6 are labelled.

It is known from the literature that superficial vein patterns are unique to each part of the body, e.g. leg, arm, feet, hands, etc. It is also known from the literature that vein patterns vary with age and sex of the individual.

Fig. 2 illustrates a series of images of superficial vein patterns on the forearm of subjects of different demographic groups. Image a1 shows a male of 19 years old. Image a2 shows a female of 16 years old. Image a3 shows a male of 47 years old. Image a4 shows a male of 72 years old. Image b 1 shows a male of 19 years old. Image b2 shows a female of 22 years old. Image b3 shows a female of 50 years old. Image b4 shows a male of 57 years old. It will be recognized that the vein pattens vary between the different demographic groups. This variation is regular and predictable to an extent that the inventors have recognized that this can be used to derive information about the individual or the area being treated.

The inventors thus propose to use the predictable variation in superficial vein patterns as a function of body part and demographics to identify the demographic category of person or body part to which the device is being applied. Currently, any hand-held devices in both personal health (e.g. shavers, hair removal devices, blood pressure sensor) and healthcare devices (e.g. CT imaging, X-Ray imaging) lack a capacity to automatically detect the body on the which the device is being used.

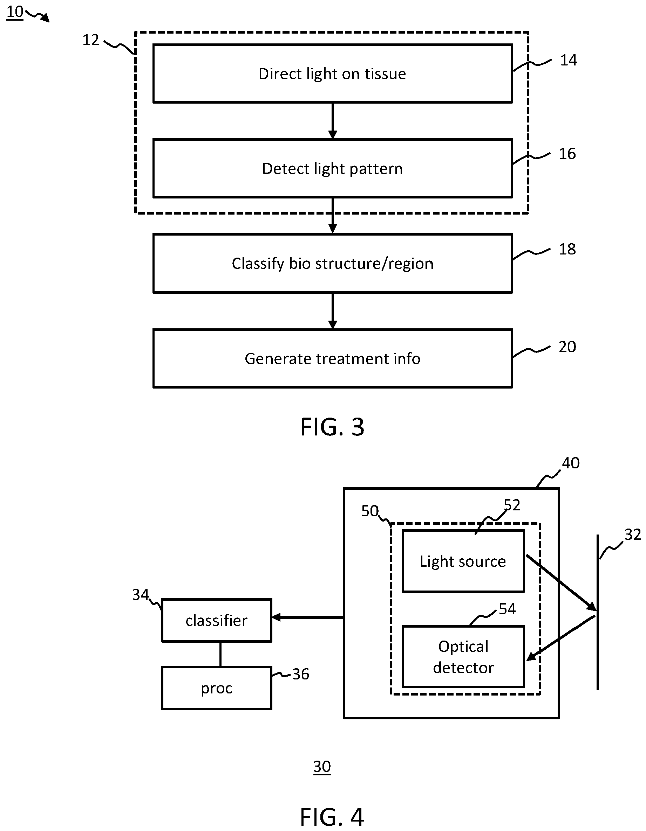

Fig. 3 outlines in block diagram form steps of an example method according to one or more embodiments. The steps will be recited in summary, before being explained further in the form of example embodiments.

Provided is an optical sensing method 10 for use during application of a body-treatment device to the body of a user.

The method comprises detecting 12 a vein pattern of a user's superficial veins. The detecting 12 comprises directing 14 light into surface tissue of the user during use of the body treatment device, and detecting 16 a pattern of light reflected from the surface tissue responsive to the directing of the light. The method further comprises applying 18 a classification module to provide one or more classifications of a biological structure or region thereof to which a vein pattern represented in the detected pattern of light belongs. The method further comprises generating 20 treatment-related information using an output from the classification module for at least one detected vein pattern.

The method may be embodied in the form of a software product, for example a computer program product.

For example, one aspect of the invention is a computer program product comprising machine-executable instructions configured, when executed by a processor, to cause the processor to:
control detection of a vein pattern of a user's superficial veins by: communicating with a light source to control the light source to direct light into surface tissue of the user during use of the body treatment device; and communicating with an optical detector to control the optical detector to detect a pattern of light reflected from the surface tissue responsive to the directing of the light;
apply a classification module to provide one or more classifications of a biological structure or region thereof to which a vein pattern represented in the detected pattern of light belongs; and
generate treatment-related information using an output from the classification module for at least one detected vein pattern.

As noted above, the method can also be embodied in hardware form, for example in the form of an optical sensing system which is configured to perform an optical sensing method such as that outlined above.

To aid understanding, Fig. 4 presents a schematic representation of an example optical sensing system 30 in accordance with one or more embodiments of the invention.

The system 30 comprises a vein visualization sub-system 40. This may be for integration in the body treatment device. The vein visualization sub-system 40 is configured to detect a pattern of a user's superficial veins.

The vein visualization subsystem 40 comprises an optical arrangement 50 for acquiring optical (e.g. image) data of a tissue patch. The optical arrangement includes a light source 52 for directing light into surface tissue 32 of the user. The optical arrangement further includes an optical detector 54 for detecting a pattern of light reflected from the surface tissue having a vein pattern represented therein.

The system further includes a classification module 34 for application to an output from the vein visualization sub-system 40 to provide one or more classifications of a biological structure or region to which a vein pattern represented in the detected pattern of light belongs.

The system further comprises a processing module 36 adapted to generate treatment-related information using an output from the classification module for at least one detected vein pattern.

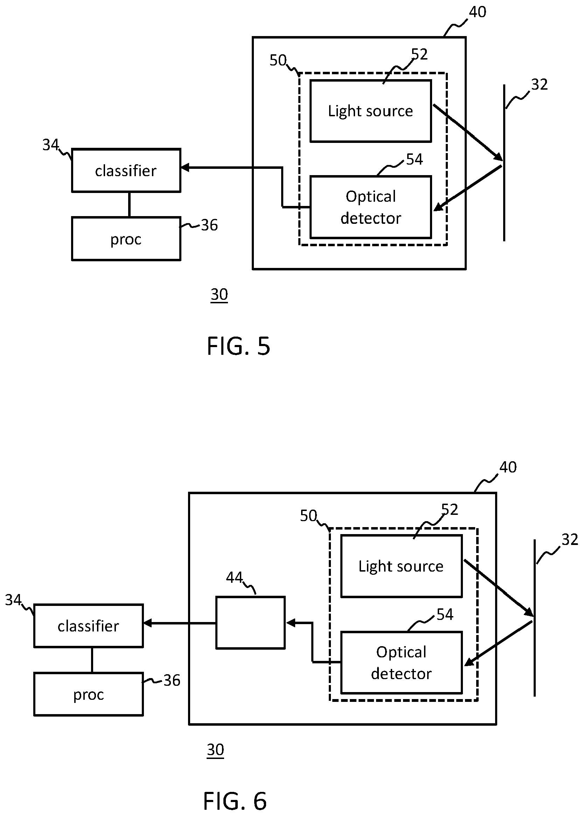

In some embodiments, the classification module 34 may be adapted to receive an output directly from the optical detector 54. For example, the classification module may receive the detected pattern of light from the optical detector and may generate the classification using the pattern of light. An example of such a configuration is illustrated in Fig. 5.

In some embodiments, the vein visualization 40 may comprise a pattern extraction module 44 for extracting a representation of a vein pattern from the detected pattern of light, and wherein the classification module 34 is adapted to operate on the extracted representation of the vein pattern. An example of such a configuration is illustrated in Fig. 6.

In any of the above examples, a controller (not shown) may be provided for controlling operation of the vein visualization subsystem, for example for controlling operation of the light source and optical detector to detect the light patterns.

In any of the above examples, although the illustrated components are depicted with a particular architecture and spatial arrangement, this may be understood as schematic only. The various components may be provided in practice in the form of a distributed system. In some embodiments, the different components may be spatially grouped in a different way to that depicted in the Figures. For example, in some embodiments, each of the classification module 34, the processing module 36, and the optional pattern extraction module 44 may be integrated in a single controller module. Furthermore, although the various components of the system are illustrated as being provided as separate units, in fact the functional operations of some or all of the components may in some embodiments be performed by a single processing device. The various modules may be software modules or hardware modules.

In some embodiments, the optical detector 54 takes the form of a camera, and the detected patten of light may be an image which is generated by the camera.

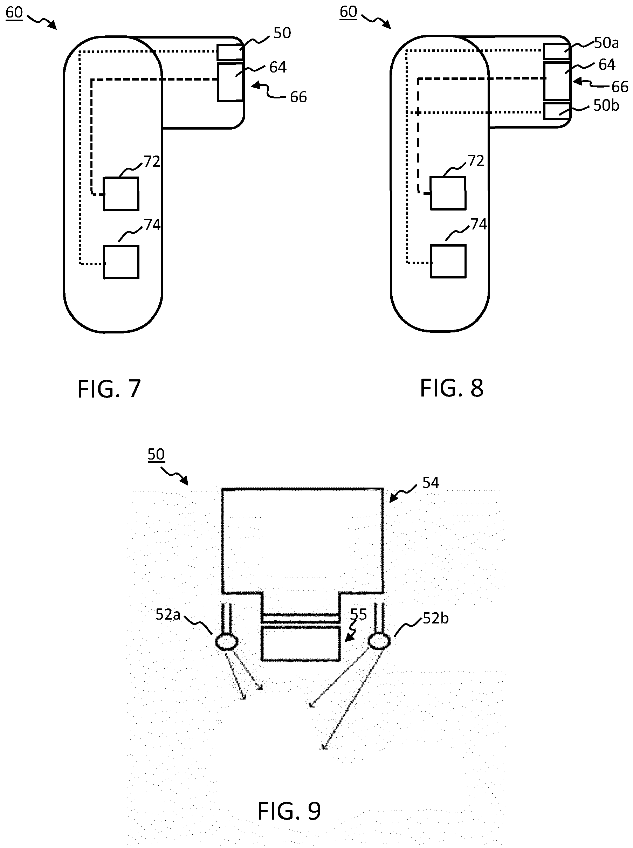

In some embodiments, the optical sensing system 30 may be integrated in a body treatment device. Two examples are schematically illustrated in Figs. 7 and 8. Each figure illustrates a body treatment device 60 comprising components of an optical sensing system integrated therein. In both examples, the body treatment device includes an operative treatment portion 64 which is adapted to perform a body treatment function. The operative treatment portion 64 defines an operative area 66 at a surface of the device for engaging with tissue to perform the body treatment function.

Referring to Fig. 7, adjacent to the operative area 66 of the body treatment portion 64 is an optical arrangement 50 of an optical sensing system 30 for acquiring a light pattern containing a representation of a vein pattern of a tissue area. An optical input/output area is provided at a surface of the device 60 arranged for being applied against the skin of the user during use of the device 60 and wherein the light source and the optical detector of the optical arrangement 50 emit light and detect light via the optical input/output area.

The operative treatment portion 64 is operatively coupled to a treatment controller 72 which is adapted to control operation of the operative treatment portion. By way of non-limiting example, the operative treatment portion may be adapted to perform a hair removal function, a hair cutting function, or an optical skin treatment function. The optical arrangement 50 is coupled to a sensing controller 74 which contains the classification module 34, the processing module 36, and the optional pattern extraction module 44. A different structural arrangement of the various components is of course possible, and the Figure provides an illustrative example only.

Fig. 8 differs from Fig. 7 in that a plurality of the optical arrangements 50 are provided, including at least one optical arrangement 50a disposed on one side of the operative area 66 and at least a second optical arrangement 50b disposed on an opposite side of the operative area 66.

Consistent with either example, the body treatment device may in some embodiments be an Intense Pulsed Light (IPL) hair removal device. Consistent with either example, the body treatment device may in some embodiments be a shaver or other mechanical hair removal device.

As an alternative to being integrated in the body treatment device, it is also possible for the optical sensing system 30 to be integrated in a separate unit or device, for example an attachment adapted to couple, e.g. removably, to a body of the body treatment device, or a wearable device, e.g. a wrist or hand or finger worn device.

Embodiments of the invention include functionality for acquiring a pattern of light reflected from the surface tissue. Implementation options in respect of this functionality will now be discussed in further detail.

As mentioned above, an optical arrangement 50 including a light source 52 and optical detector 54 may be employed.

With regards to the light source 52, this may comprise one or more lighting elements, each adapted to generate a light output for incidence upon a tissue surface of a user, for example during use of the body treatment device. In some embodiments, the lighting elements may be adapted to generate a light output in the near infrared (NIR) or infrared (IR) frequency range. In some embodiments, each lighting element may be an LED lighting element. The light source may comprise a bank of LEDs.

By way of example, Infrared Emitting Diodes (IREDs) are solid state light sources which generate a light output in the near infrared part of the spectrum. IREDs allow for cheap, efficient production of near infrared light with only a small spatial footprint.

As light penetrates the body tissue, it is reflected by the hemoglobin in the blood, thereby providing a reflected light pattern which reflects the shape and structure of the subject's superficial vascular pattern. Near infrared (NIR) and infrared (IR) light are particularly well reflected by hemoglobin.

To capture the reflected light pattern, an optical detector 54 is provided. In a simple case, this may comprise a 2D photodetector array or similar. In some further examples, the optical detector may be provided in the form of a camera, such that detected pattern of light takes the form of an image acquired by the camera.

In some embodiments, the optical detector make take the form of a charge coupled device (CCD). A CCD is a solid state electrical device capable of converting light into an electronic signal. A CCD provides a form of image sensor. It comprises an array of light sensitive elements (each referred to as a pixel), and wherein each light sensitive element stores a charge proportional to the amount of light which is received over a certain measurement interval. In effect, a photon of light which falls within the area defined by one of the pixels is converted into one (or more) electrons and the number of electrons collected is directly proportional to the intensity of the scene at each pixel. The charge stored in each pixel is shifted through a series of gates to a readout register, where it is converted into a voltage signal that can be digitized and processed. The signals from the collection of pixels can be used to build up an image of the scene of interest. The term "charged-coupled" refers to the coupling of electrical potentials that exist within the chemical structure of the silicon material that make up the layers of the chip.

Since the charge in each pixel is shifted in sequence, CCDs can produce high-quality images with low noise and high dynamic range. CCDs can be small in size, simple in operation and easy to manufacture. This makes them ideal for integration in hand-held devices.

CCDs can be configured to be sensitive at a wide variety of different wavelengths, ranging from about 0.1 nm (soft x-ray) to around 400 nm (blue visible) through to about 1000 nm (near infrared) with a peak sensitivity at around 700 nm. For purposes of embodiments of the present invention, sensitivity across a near infrared (NIR) or Infrared (IR) range may be preferred, although other wavelengths may also be used.

According to some examples, the light source 52 comprises an array of lighting elements, for example an elongate or linear array of lighting elements.

In some embodiments, optical detector 54 may comprise an array of light detection elements, for example an elongate or a linear array of light detection elements. For example, if a CCD camera is used, the pixels of the camera may form an elongate or a linear array. This may be a ID linear array or, more preferably, may be 2D strip of pixels.

In some embodiments, light source comprises an elongate or linear array of lighting elements and the optical detector comprises an elongate or linear array of light detection elements being parallel with the elongate or linear array of lighting elements. This provides a structurally and functionally efficient configuration.

By way of one example, Fig. 9 illustrates a configuration for an optical arrangement 50 according to one or more embodiments. The optical arrangement 50 includes a light source which comprises a first array 52a of NIR or IR LEDs and a second array 52b of NIR or IR LEDs and includes an optical detector 54 which comprises an NIR or IR sensitive CCD. Optionally between the CCD and the tissue surface being irradiated may be disposed an NIR or IR light filter 55 to restrict the light input to the CCD to only the NIR or IR range.

Light generated by the bank of LEDs penetrates the skin of the subject and is reflected by the hemoglobin in the blood present in the superficial veins. The reflected light may pass through the optional IR or NIR filter before being captured by the CCD camera.

In some embodiments, the vein visualization sub-system 40 may further comprise a proximity sensor for detecting a proximity distance between the optical detector 54 and the surface tissue of the user. There may further be included a patch normalization module for applying a positive or negative enlargement of the detected light pattern of a magnitude determined in dependence on the sensed proximity distance in order to normalize each detected light pattern size to a standardized size.

As mentioned above, in some embodiments, the vein visualization subsystem 40 may further comprises a pattern extraction module 44 for extracting a representation of a vein pattern from the detected pattern of light before application of the classification module 34. The classification module in this case is adapted to operate on the extracted representation of the vein pattern. This may be preferred in some embodiments, since the extraction of the vein pattern may ensure greater consistency in the classification and may make the classification process more reliable or straightforward.

In some embodiments, the representation of the vein pattern comprises a vein pattern mask. In some embodiments, the pattern extraction module may be a trained neural network which is trained to extract the vein pattern mask from a detected light pattern. Other example implementations to extract the vein patten may include more classical image processing or machine vision algorithms such as edge detection, outline detection, thresholding-based segmentation, region growing, skeletonization and feature-based detection.

With regards to the option of applying a neural network to extract a vein pattern mask from the detected pattern of light (e.g. detected image), this may for example be achieved by training a convolutional neural network. The training data for the neural network may comprise a plurality of sample detected light patterns (e.g. images) containing reflected representations of vein patterns, the light patterns having the same format as the output from the optical detector, and wherein each sample detected light pattern is annotated with a ground truth vein pattern mask which has been extracted from the image in advance, e.g. manually by a human operator or automatically via a different image processing tool or even another neural network being used for transfer learning.

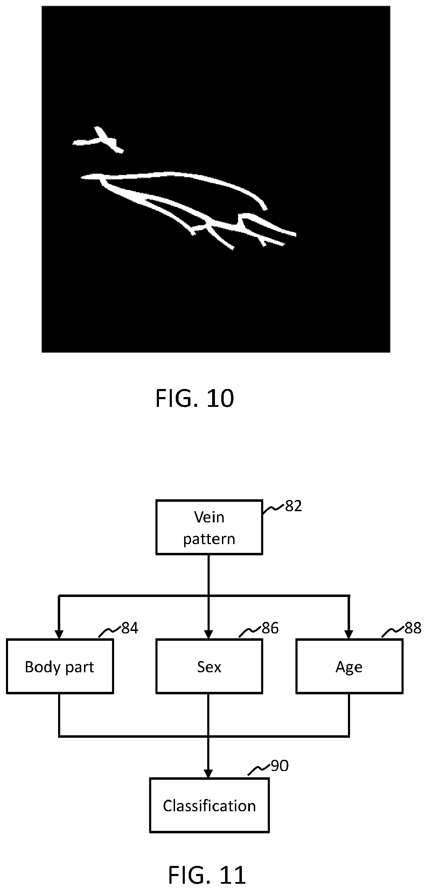

Fig. 10 depicts an example vein pattern mask extracted from an acquired light pattern (e.g. image).

Reference is made for example to the project "Near-Infrared-to-Visible Vein Imaging via Convolutional Neural Networks and Reinforcement Learning project", which is located at the following web address as of March 2023: https://sithub.com/cviaai/VEINCV-RL. This approach uses image segmentation along with Reinforcement Learning to determine the mask of the veins.

Embodiments of the invention include functionality for providing one or more classifications of a biological structure or region to which a vein pattern represented in the detected pattern of light belongs. The system may include a classification module for performing this functionality.

By way of example, the one or more classifications generated by the classification module may include one or more of: an identification of a body part to which the vein pattern belongs; a classification of one or more demographic characteristics of the individual to which the vein pattern belongs, for example age and/or sex; and/or an identification of a unique skin patch within which the vein pattern is situated.

According to one set of embodiments, it is proposed that the classification module comprise a trained neural network.

For example, the trained neural network may be a network trained using training data which comprises vein pattern masks annotated with ground truth information indicative of one or more of: a body part to which the vein pattern belongs, an age of the individual to which the vein pattern belongs; a sex of the individual to which the vein pattern belongs, and a unique skin patch identifier of the skin patch to which the vein pattern belongs.

In some embodiments, a method is applied which involves a training procedure for training the neural network comprising training the network using training data which comprises vein pattern masks annotated with ground truth information indicative of a biological structure or region to which a vein pattern represented in the vein pattern mask belongs. For example, the ground truth information may be indicative of one or more of: a body part to which the vein pattern belongs, age of the individual to which the vein pattern belongs, a sex of the individual to which the vein pattern belongs, and a unique skin patch identifier of the skin patch to which the vein pattern belongs.

In some embodiments, the method may further comprise quantizing the neural network after training.

The neural network may be quantized before integration in the device to reduce the size of the model, thereby enabling it to be deployed by hardware having small memory and processing capacity.

In some embodiments, the classification module may be for integration within the body treatment device and may comprise a memory storing the neural network and a processor for controlling application of the neural network.

The method of training may be provided as a separate aspect of the invention, or may be provided as part of the optical sensing method. For example, a method of training an artificial neural network may comprise receiving training data, wherein the training data comprises a plurality of training data entries, each training data entry comprising a vein pattern mask annotated with ground truth information indicative of a biological structure or region to which a vein pattern represented in the vein pattern mask belongs; and training the artificial neural network using the training data.

More detail will now be presented regarding the training of a suitable neural network model for generating the classification of a biological structure or region to which a vein pattern represented in the detected pattern of light belongs.

With regards to the generating of the training data set, the following considerations may be taken into account. The plurality of sample vein pattern masks comprised in the training dataset may correspond to light patterns acquired from a population of people which is diverse in demographics, in order to ensure the dataset has varied samples. In some embodiments, image augmentation may be applied to the acquired light patterns before extraction of the vein pattern mask to account for images taken in different orientations and lighting condition.

With regards to the neural network, in accordance with some embodiments, it is proposed to use a convolutional neural network (CNN) with U-net architecture.

A convolutional neural network (CNN) is an example of Deep Learning. Image classification involves the extraction of features from an input image and the detection of patterns in the dataset between extracted features and certain classification class labels. Through the training process, the CNN learns the patterns associated with each class.

One approach to training a CNN for classifying the vein pattern masks might be as follows. An initial CNN model with U-net architecture may undergo preliminary training using a pre-existing image dataset such as the COCO (Common Objects in Context) dataset. U-net is a widely used architecture for bio-medical image classification.

Following the preliminary training, further training may be performed using the dedicated training dataset discussed above comprising vein pattern images annotated or labelled with one or more classifications. A simple approach is to train a single respective model to perform each single classification which is desired. For example, if it is desired for the classification module to classify each of age of the user, sex of the user, and the body part, then three separate models may be trained, each trained to generate one of the respective classifications.

Fig. 11 schematically illustrates the basic information flow achieved through use of the classification module 34.

A representation of a vein pattern 82, e.g. a vein pattern mask or the acquired light pattern, is provided as input to the classification module. In the present example, three trained neural networks are applied to the input vein pattern: a first neural network 84 trained to classify a body part to which the vein pattern belongs, a second neural network 86 trained to classify a sex/gender of the individual to which the vein pattern belongs; and a third neural network 88 trained to classify an age of the individual to which the vein pattern belongs. The combination of outputs from these three neural networks provides a composite classification 90 of the combination of body part, age and sex/gender.

Of course, it is not essential to provide a multiplicity of neural networks. In further embodiments, just a single neural network may be provided which is trained to generate a single classification, e.g. one of age, sex and body part, or trained to generate a plurality of such classifications.

As will be discussed in more detail later, another example classification which a neural network may be trained to generate is an identification of a unique skin patch within which the vein pattern is situated. This can allow for navigation/localization functionality for example.

As discussed above, embodiments of the present invention include functionality for generating treatment-related information using an output from the classification module for at least one detected vein pattern. There are different options for the type of treatment-related information which is generated.

In some embodiments, the treatment related information comprises a setting for an operational parameter of the body treatment device.

For example, in some embodiments, the treatment related information comprises electronic control instructions for adjusting an operational parameter of the body treatment device in dependence upon the classification.

The system may include a memory module which stores a look-up table permitting mapping between classifications and corresponding operational parameter settings. The particular mappings between classifications and settings may be pre-defined and/or may be configurable by a user.

For example, by providing the ability to auto detect the body part and/or user demographics, functionality can be provided permitting self-adjustment of operational settings of the body treatment device without the need for external sensors or human intervention. This provides an improved experience to the user and also avoids the problem of incorrect settings being applied by a user. In the case for example of an optical hair removal device, incorrect settings with regards to light intensity could be hazardous.

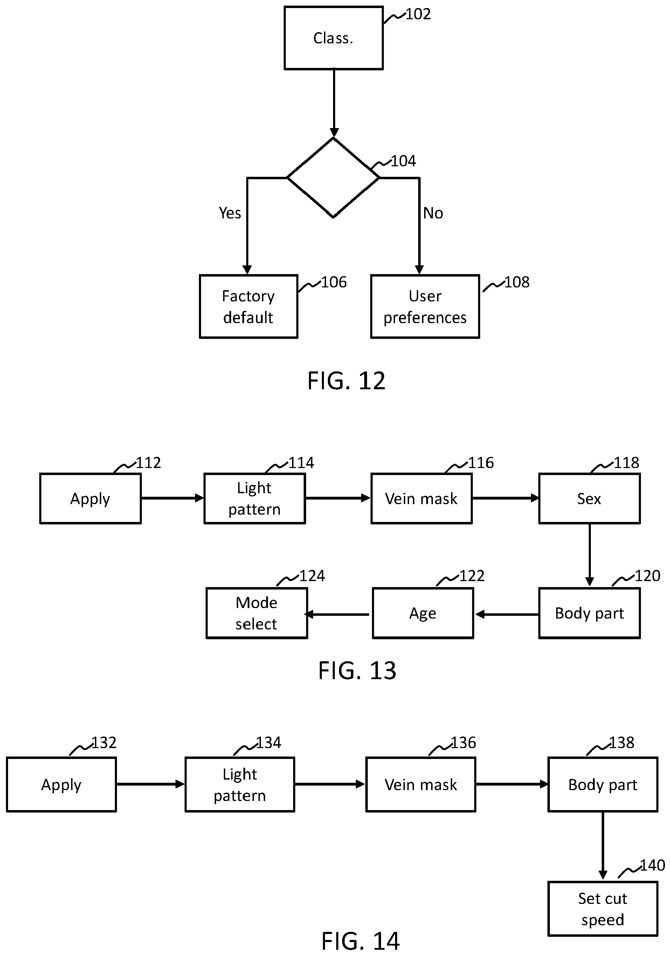

Fig. 12 schematically outlines an example processing logic in accordance with one or more embodiments in which a classification generated by the classification module 34 is used to determine an operational parameter setting for the body treatment device. A classification 102 is generated by the classification module. By way of example, the classification may comprise a classification of one or more of: an identification of a body part to which the vein pattern belongs; a classification of the age of the individual to which the vein pattern belongs, a classification of the sex or gender of the individual to which the vein pattern belongs. The classification may consist of a single one of such classifications or a composite classification comprising more than one. In the illustrated example, in a decision step 104, it is determined whether custom user-defined settings exist in respect of the mapping between classifications and operation parameter settings. If not, factory default mappings 108 are used. If so, then user-defined preferences 106 for the mappings are used. User-defined settings may be configurable via a user interface for example. They may be configurable via an associated software application.

To further illustrate the concept of auto-configuring operational parameter settings based on the vein visualization, some further implementation examples will now follow.

Fig. 13 illustrates an example processing flow according to a first example implementation for enabling a multi-utility body treatment device. A multi-utility body treatment device means a body treatment device which is switchable between a plurality of different body treatment modes, each mode performing a different type or class of body treatment. For example, the device may be operable in each of a mechanical shaving mode and an optical hair removal mode. The workflow comprises a user applying 112 the device to a body part; the optical arrangement acquiring 114 a light pattern or image representation of a vein pattern of the body part; extracting 116 a vein pattern mask form the representation; classifying 118 a sex of the user by applying a trained classifier to the vein pattern mask, such as a trained neural network, classifying 120 a body part to which the vein pattern belongs by applying a trained classifier to the vein pattern mask, such as a trained neural network; classifying 122 an age of the user by applying a trained classifier to the vein pattern mask, such as a trained neural network; generating 124 control instructions for selecting an operational mode of the body treatment device in dependence upon the classification, for example based on a lookup table.

For example, the system may be configured to select an optical hair removal mode if the user is female and a mechanical hair removal mode is the user is male. By way of further example, the system may select a mechanical hair removal mode if the body part is the face, or an optical hair removal mode if the body part is a leg.

According to some embodiments, the device may be applicable to multiple different body parts and wherein a setting of the device is varied in dependence upon the body part. For example a shaver might be used on the face, chest or armpit.

Fig. 14 illustrates an example processing flow according to an example implementation in which the body treatment device is a shaving device and wherein the operational parameter comprises a cutting speed/rate of a cutting mechanism of the shaving device. For example, a higher cutting rate may be set when the device is being applied to the face compared to when the device is being applied to the chest. For example, a user might prefer higher RPM of the shaver blade for beard cutting, and might prefer a lower RMP for cutting chest hair.

The workflow comprises a user applying 132 the device to a body part; the optical arrangement acquiring 134 a light pattern or image representation of a vein pattern of the body part; extracting 136 a vein pattern mask form the representation; classifying 138 a body part to which the vein pattern belongs by applying a trained classifier to the vein pattern mask, such as a trained neural network; and generating 140 control instructions for selecting setting a cutting rate/speed of the shaver in dependence upon the classification, for example based on a lookup table.

According to some embodiments, the body care device may be an Intense Pulsed Light (IPL) hair removal device. Such hair removal devices permit adjustment of optical characteristics of the generated treatment light, for example adjustment of a set intensity level of light. This may be adjusted according to body part and/or age for example.

Thus, in some embodiments, the operational parameter comprises an intensity level of pulsed light generated by the device.

Fig. 14 illustrates an example processing flow according to an example implementation in which the body treatment device is an Intense Pulsed Light (IPL) hair removal device and wherein the operational parameter comprises an intensity level of pulsed light generated by the device.

The workflow comprises a user applying 152 the device to a body part; the optical arrangement acquiring 154 a light pattern or image representation of a vein pattern of the body part; extracting 156 a vein pattern mask form the representation; classifying 158 a body part to which the vein pattern belongs by applying a trained classifier to the vein pattern mask, such as a trained neural network; classifying 160 an age of the user by applying a trained classifier to the vein pattern mask, such as a trained neural network and generating 162 control instructions for configuring an intensity level of light generated by the device in dependence upon the classification, for example based on a lookup table.

With regards to age dependency, the intensity level may be adjusted in dependence upon the age of the user, since older users have more fragile skin compared to younger users. Thus, an intensity level may be set lower for older users.

The optical sensing system 30 also has utility for obtaining performance or monitoring information pertaining to one or more usage/treatment sessions. In particular, according to an advantageous set of embodiments, the optical sensing system includes functionality for tracking body coverage with the body treatment device by using the classifier to uniquely identify a skin patch being scanned and, by aggregating skin patch detections over one or more treatment sessions, track the overall body coverage by the device.

By way of one non-limiting example, when using an optical hair removal device, such as an IPL device, to achieve best results, it is best to apply the device to the body evenly and in a regular non overlapping pattern. Any gaps in application of the device results in inferior hair removal. At the same time, overlapping a same area multiple times may lead to overexposure.

Therefore, it would be advantageous to provide functionality to guide uniform application. This applies also for any other body treatment devices where even, consistent application is desirable.

Thus, in accordance with the presently described set of embodiments, the detection of superficial vein patterns is used to track device coverage over the body.

The same concept also allows for reliable and precise collection of exposure statistics, which is beneficial both for guiding optimal use of the device as well as for assisting in lifetime-based replacement of consumables, such as a head of the device.

Fig, 16 outlines components of an example system according to one or more embodiments in which coverage tracking is performed.

The system may be the same as the system of any of the embodiments for example of Fig. 4, Fig. 5, or Fig. 6 except for at least the following further characteristics.

The previously discussed classifier module 34 in this set of embodiments is adapted to generate a classification comprising an identification of a unique skin patch within which the vein pattern is situated.

In addition, the system 30 further comprises a coverage tracking module 46 comprising one or more processors adapted to, during a body treatment session: receive an indicator of each detected unique skin patch; and store a record of each skin patch detection in a memory module (38) to compile a body treatment session history.

In this set of embodiments, the treatment-related information generated by the processing module may comprise the aforementioned body treatment session history. Additionally or alternatively, the treatment related information may include analytics information derived from the body treatment session history.

For example, in some embodiments, the processing module 36 may be adapted to identify from the treatment session history any unique skin patches for which multiple detections are recorded in the session history and wherein the treatment-related information includes an indication of each unique skin patch for which multiple detections are recorded. This provides an indication of skin areas which have been covered more than once in the treatment session. Detecting such areas is valuable since covering areas more than once can cause issues arising from overexposure.

In addition to or instead of detecting skin patches which have been covered more than once in a given session, it may also be valuable to detect skin patches which have not been covered at all. To this end, a data structure such as a database may be provided which records a target set of unique skin patches to be covered in each treatment session of at least a given session type, and wherein the skin patches recorded in the treatment session history is compared against the target set of patches to identify patches which have not been covered in the treatment session.

To this end, in some embodiments, the processing module 36 may be comprised by an analytics module. The analytics module may be adapted to retrieve the body treatment session history for at least one body treatment session from the memory module. The analytics module may be further adapted to access a data structure recorded in a datastore which records a set of known or assumed unique skin patches comprised by the user (i.e. a target set of skin patches of the user). The treatment-related information in this case may comprise coverage feedback indicative of coverage and/or non-coverage of different of the unique skin patches during the treatment session.

The aforementioned data structure recorded in the datastore may comprise a body map comprising an indicator of a bodily location of each of the set of unique skin patches.

In some embodiments, the coverage feedback may comprise a graphical representation of the body map, including a visual indication of coverage and/or non-coverage of each of the unique skin patches, and optionally wherein the processing module is adapted to communicate the coverage feedback to a user interface or to a mobile computing device.

There may be provided functionality for implementing a calibration procedure for determining the aforementioned set of known or assumed unique skin patches comprised by the user (i.e. the target set of skin patches of the user). This process may comprise following instructions to apply the optical arrangement of the vein visualization subsystem onto a plurality of different skin patch locations and wherein the subsystem acquires a light patterns or image of the superficial veins at each location. From this, a reference vein pattern mask is acquired for each of the defined locations and these may form the set of known skin patches comprised by the user. This can be used to form the body map, since each vein pattern acquired in the visualization process can be associated with a known location.

In an advantageous set of embodiments, the treatment related information, for example the coverage information, is communicated to a secondary device comprising a user interface for presenting the information to a user, or may be communicated to a server, for example a cloud-based server to facilitate access of the information from a portable computing device.

According to an advantageous set of embodiments, the treatment-related information is received by a software application installed on a mobile computing device, and wherein the software application controls a user interface of the mobile computing device to provide a representation of the information.

For example, the software application may present a visual representation of the aforementioned body map showing the coverage and/or non-coverage of each of the unique skin patches. The software application may permit a user to display coverage information for a selected one of a plurality of different treatment sessions for which a treatment history is recorded, e.g. corresponding to different days. The software application may generate recommendations for the user based on the coverage information, e.g. to guide the user in providing more uniform and complete coverage.

For implementation, it may be preferred to integrate the optical arrangement in the body treatment device. A preferred embodiment may be the use of the aforementioned parallel light source array and optical detector array to thereby effectively provide a parallel light injecting strip and scanning strip. Additionally, it may be advantageous to incorporate the previously discussed optional proximity sensor for measuring a proximity distance of the optical detector to the skin surface, and performing normalization of each scanned patch to a uniform size. By using this configuration, when the device is pulled along a certain area, the vein visualization subsystem will scan a pattern of blood vessels in the underlying tissue and other possible markers, and these can be passed to the pattern extraction module and/or the classification module.

In accordance with some embodiments, a respective pair of light injecting and scanning strips may be included at two ends of an operative treatment head of the device, e.g. at both ends of the head, such that scanning can be performed of an area both before and after exposure. This may make the system less sensitive to a direction in which a user moves the head.

In accordance with one or more embodiments, additional functionality may be provided for analyzing the light patterns detected by the optical detector (e.g. images) and/or analyzing vein pattern representations (e.g. masks) extracted from the detected light patterns with a diagnostic analysis module. In particular, it has been recognized by the inventors that the optical information acquired by the optical detector during the course of operation could efficiently serve a secondary function of skin health or skin pathology assessment. For example, in some embodiments, the optical sensing system further comprises a diagnostic analysis module and wherein the diagnostic analysis module is adapted to receive the pattern of light detected by the optical detector and/or a vein pattern mask extracted from the detected pattern of light, and to determine one or more diagnostic indicators or generate one or more diagnostic classifications. By way of one example, the diagnostic analysis module may be adapted to process an extracted vein patten mask to derive a varicose vein diagnostic classification, e.g. present/absent and/or a degree of severity. By way of a further example, the diagnostic analysis module may be adapted to receive a pattern of light (e.g. image) detected by the optical detector and detect or classify a skin lesion. For example, mole detection and/or classification may be performed.

In some embodiments, the information derived by the diagnostic analysis module may be used in further configuring one or more operational parameters of the device. For example, the processing module may be adapted to adjust an intensity of treatment light applied responsive to detection by the diagnostic analysis module of a skin lesion falling within any of a pre-defined set of classifications. For example, where a mole is detected, intensity may be reduced.

Additionally or alternatively, information derived by the diagnostic analysis module may be used to provide additional feedback to a user or healthcare provider, for example through a user interface integrated in the body treatment device or optical sensing system, or through a secondary computing device such as an app installed on a mobile computing device. By way of example, the processing module may be adapted to generate treatment advice feedback for a user in dependence upon a type and/or severity of skin lesion which is detected. For example, responsive to detecting a skin lesion, advice may be generated indicating a treatment dependent on the class of lesion detected, e.g. application of topical medicaments such as cream. In this context, 'skin lesion' is taken to include any abnormal pathology of the skin, including wounds, rashes, moles, inflammation, abscesses and so on.

With regards to implementation, the diagnostic analysis module may employ a machine learning algorithm to generate the diagnostic information from the light patterns and/or the extracted vein masks, or may employ a more classical type of machine vision algorithm such as image segmentation, feature extraction, or pattern recognition. With regards to the option of using a machine learning algorithm, one possibility is to train an artificial neural network (ANN), for example a convolutional neural network, to generate diagnostic classifications based on input light patterns (or images) or extracted vein pattern masks. In some examples, there may be a separate ANN model trained for detection or classifying each different type of skin pathology of interest.

Embodiments of the optical sensing system described in the document can be applied to a variety of different use cases. Although the examples discussed above have related primarily to use with body treatment devices, the same inventive concept may be applied for substantially any handheld device which is applied to the body during use, for example where localization on the body surface may be useful. This may include for example handheld medical scanning devices such as ultrasound probes, or other body-applied measurement devices. Another possible application area is for use in hair counting, where is may be useful to perform localization of skin patches covered by each of a series of acquired images, for example with respect to a full head map.

Various embodiments described above employ use of a trained artificial neural network. The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, (weightings of) the mathematical operation of each neuron may be modified until the error converges. Known methods of modifying a neural network include gradient descent, backpropagation algorithms and so on.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. An optical sensing system (30) for a body treatment device, comprising:
a vein visualization sub-system (40) for integration in the body treatment device, for detecting a pattern of a user's superficial veins, comprising an optical arrangement (50) which includes
a light source (52) for directing light into surface tissue of the user during use of the body treatment device, and
an optical detector (54) for detecting a pattern of light reflected from the surface tissue having a vein pattern represented therein;
a classification module (34) for application to an output from the vein visualization sub-system to provide one or more classifications of a biological structure or region to which a vein pattern represented in the detected pattern of light belongs; and
a processing module (36) adapted to generate treatment-related information using an output from the classification module for at least one detected vein pattern.

2. The system of claim 1, wherein the one or more classifications generated by the classification module comprise:
an identification of a body part to which the vein pattern belongs;
a classification of one or more demographic characteristics of the individual to which the vein pattern belongs, for example age and/or sex; and/or
an identification of a unique skin patch within which the vein pattern is situated.

3. The system of any of claims 1-2, wherein the vein visualization subsystem further comprises a pattern extraction module for extracting a representation of a vein pattern from the detected pattern of light; and wherein the classification module is adapted to operate on the extracted representation of the vein pattern, and optionally wherein the representation of the vein pattern comprises a vein pattern mask.

4. The system of claim 3, wherein the representation of the vein pattern comprises a vein pattern mask, and wherein the pattern extraction module is a trained neural network which is trained to extract the vein pattern mask from a detected light pattern.

5. The system of any of claims 1-4, wherein the classification module comprises a trained neural network, and
optionally wherein the trained neural network is a network trained using training data which comprises vein pattern masks annotated with ground truth information indicative of one or more of:
a body part to which the vein pattern belongs,
age of the individual to which the vein pattern belongs,
sex of the individual to which the vein pattern belongs, and
a unique skin patch identifier of the skin patch to which the vein pattern belongs.

6. The system of any of claims 1-5, wherein the treatment related information comprises electronic control instructions for adjusting an operational parameter of the body treatment device in dependence upon the classification, and
optionally, wherein:
the operational parameter comprises selection between available treatment modes, wherein a first mode is a shaving mode, and a second mode is an optical hair removal mode; or
the body treatment device is a shaving device and wherein the operational parameter comprises a cutting speed/rate of a cutting mechanism of the shaving device; or
the body treatment device is an Intense Pulsed Light (IPL) hair removal device and wherein the operational parameter comprises an intensity level of pulsed light generated by the device.

7. The system (30) of any preceding claim, wherein
the classifier module (34) is adapted to generate a classification comprising an identification of a unique skin patch within which the vein pattern is situated, and
the system further comprises a coverage tracking module (46) comprising one or more processors adapted to, during a body treatment session,
receive an indicator of each detected unique skin patch; and
store a record of each skin patch detection in a memory module (38) to compile a body treatment session history.

8. The system of claim 7, wherein the processing module is adapted to identify from the treatment session history any unique skin patches for which multiple detections are recorded in the session history and wherein the treatment-related information includes an indication of each unique skin patch for which multiple detections are recorded.

9. The system of claim 7 or 8, wherein the processing module is comprised by an analytics module,
wherein the analytics module is adapted to retrieve the body treatment session history for at least one body treatment session from the memory module;
wherein the analytics module is further adapted to access a data structure recorded in a datastore which records a set of known or assumed unique skin patches comprised by the user; and
wherein the treatment-related information comprises coverage feedback indicative of coverage and/or non-coverage of different of the unique skin patches during the treatment session.

10. The system of any preceding claim wherein the vein visualization sub-system further comprises
a proximity sensor for detecting a proximity distance between the optical detector and the surface tissue of the user and
a patch normalization module for applying a positive or negative enlargement of the detected light pattern of a magnitude determined in dependence on the sensed proximity distance in order to normalize each detected light pattern size to a standardized size.

11. A body-treatment device comprising the optical sensing system of any preceding claim.

12. An optical sensing method for use during application of a body-treatment device to the body of a user, comprising:
detecting a vein pattern of a user's superficial veins, wherein the detecting comprises:
directing light into surface tissue of the user during use of the body treatment device; and
detecting a pattern of light reflected from the surface tissue responsive to the directing of the light;
applying a classification module to provide one or more classifications of a biological structure or region thereof to which a vein pattern represented in the detected pattern of light belongs; and
generating treatment-related information using an output from the classification module for at least one detected vein pattern.

13. The method of claim 12, wherein the classification module comprises a trained neural network, and wherein the method includes a training procedure for training the neural network comprising training the network using training data which comprises vein pattern masks annotated with ground truth information indicative of one or more of:
a body part to which the vein pattern belongs,
age of the individual to which the vein pattern belongs,
sex of the individual to which the vein pattern belongs, and
a unique skin patch identifier of the skin patch to which the vein pattern belongs.

14. A computer program product comprising machine-executable instructions configured, when executed by a processor, to cause the processor to:
control detection of a vein pattern of a user's superficial veins by:
communicating with a light source to control the light source to direct light into surface tissue of the user during use of the body treatment device; and
communicating with an optical detector to control the optical detector to detect a pattern of light reflected from the surface tissue responsive to the directing of the light;
applying a classification module to provide one or more classifications of a biological structure or region thereof to which a vein pattern represented in the detected pattern of light belongs; and
generating treatment-related information using an output from the classification module for at least one detected vein pattern.

15. A method of training an artificial neural network comprising:
receiving training data, wherein the training data comprises a plurality of training data entries, each training data entry comprising a vein pattern mask annotated with ground truth information indicative of a biological structure or region to which a vein pattern represented in the vein pattern mask belongs; and
training the artificial neural network using the training data.
